(19) Europäisches Patentamt / European Patent Office / Office européen des brevets

(11) **EP 2 359 285 B1**

(12) **EUROPEAN PATENT SPECIFICATION**

(45) Date of publication and mention of the grant of the patent:
**11.01.2017 Bulletin 2017/02**

(21) Application number: **09796060.3**

(22) Date of filing: **18.11.2009**

(51) Int Cl.:
*G06F 19/00* (2011.01)   *G01N 33/68* (2006.01)

(86) International application number:
**PCT/IB2009/007719**

(87) International publication number:
**WO 2010/058295 (27.05.2010 Gazette 2010/21)**

(54) **NON-INVASIVE IN VITRO METHOD FOR QUANTIFYING LIVER LESIONS**

NICHTINVASIVES IN-VITRO-VERFAHREN ZUR QUANTIFIZIERUNG VON LEBERSCHÄDEN

MÉTHODE IN VITRO NON INVASIVE PERMETTANT DE QUANTIFIER DES LÉSIONS HÉPATIQUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.11.2008 US 115677 P**

(43) Date of publication of application:
**24.08.2011 Bulletin 2011/34**

(73) Proprietors:
• **UNIVERSITE D'ANGERS**
  **49035 Angers Cedex 01 (FR)**
• **Centre Hospitalier Universitaire d'Angers**
  **49100 Angers (FR)**

(72) Inventors:
• **CALES, Paul**
  **F-49240 Avrille (FR)**
• **AUBE, Christophe**
  **F-49000 Angers (FR)**

(74) Representative: **Icosa**
**83 avenue Denfert-Rochereau**
**75014 Paris (FR)**

(56) References cited:
• MAOR Y ET AL: "Improving estimation of liver fibrosis using combination and newer noninvasive biomarker scoring systems in hepatitis C-infected haemophilia patients." HAEMOPHILIA : THE OFFICIAL JOURNAL OF THE WORLD FEDERATION OF HEMOPHILIA NOV 2007 LNKD- PUBMED:17973848, vol. 13, no. 6, November 2007 (2007-11), pages 722-729, XP002596694 ISSN: 1351-8216
• HALFON P ET AL: "[Serum markers of non-invasive fibrosis in chronic hepatitis C virus infection]" LA REVUE DE MÉDECINE INTERNE / FONDÉE ... PAR LA SOCIÉTÉ NATIONALE FRANCAISE DE MÉDECINE INTERNE OCT 2006 LNKD- PUBMED:16815598, vol. 27, no. 10, October 2006 (2006-10), pages 751-761, XP002596695 ISSN: 0248-8663
• LEROY ET AL: "Prospective comparison of six non-invasive scores for the diagnosis of liver fibrosis in chronic hepatitis C" JOURNAL OF HEPATOLOGY, MUNKSGAARD INTERNATIONAL PUBLISHERS, COPENHAGEN, DK LNKD- DOI:10.1016/J.JHEP.2006.12.013, vol. 46, no. 5, 7 April 2007 (2007-04-07), pages 775-782, XP022024083 ISSN: 0168-8278
• SEBASTIANI GIADA ET AL: "Sequential algorithms combining non-invasive markers and biopsy for the assessment of liver fibrosis in chronic hepatitis B." WORLD JOURNAL OF GASTROENTEROLOGY : WJG 28 JAN 2007 LNKD- PUBMED:17278217, vol. 13, no. 4, 28 January 2007 (2007-01-28), pages 525-531, XP002596696 ISSN: 1007-9327

**(Cont. next page)**

- BOURLIERE M ET AL: "Optimized stepwise combination algorithms of non-invasive liver fibrosis scores including Hepascore in hepatitis C virus patients." ALIMENTARY PHARMACOLOGY & THERAPEUTICS 15 AUG 2008 LNKD- PUBMED:18498446, vol. 28, no. 4, 15 August 2008 (2008-08-15), pages 458-467, XP002596697 ISSN: 1365-2036
- HALFON PHILIPPE ET AL: "Comparison of test performance profile for blood tests of liver fibrosis in chronic hepatitis C." JOURNAL OF HEPATOLOGY MAR 2007 LNKD-PUBMED:17156890, vol. 46, no. 3, March 2007 (2007-03), pages 395-402, XP002596698 ISSN: 0168-8278
- MAOR Y ET AL: "Improving estimation of liver fibrosis using combination and newer noninvasive biomarker scoring systems in hepatitis C-infected haemophilia patients", HAEMOPHILIA, BLACKWELL SCIENCE, OXFORD, GB, vol. 13, no. 6, 1 November 2007 (2007-11-01), pages 722-729, XP002596694, ISSN: 1351-8216, DOI: 10.1111/J.1365-2516.2007.01548.X
- HALFON P ET AL: "[Serum markers of non-invasive fibrosis in chronic hepatitis C virus infection]", REVUE DE MEDECINE INTERNE, CMR, ASNIERES, FR, vol. 27, no. 10, 1 October 2006 (2006-10-01), pages 751-761, XP002596695, ISSN: 0248-8663, DOI: 10.1016/J.REVMED.2006.03.037
- LEROY ET AL: "Prospective comparison of six non-invasive scores for the diagnosis of liver fibrosis in chronic hepatitis C", JOURNAL OF HEPATOLOGY, ELSEVIER, AMSTERDAM, NL, vol. 46, no. 5, 7 April 2007 (2007-04-07), pages 775-782, XP022024083, ISSN: 0168-8278, DOI: 10.1016/J.JHEP.2006.12.013

- SEBASTIANI GIADA ET AL: "Sequential algorithms combining non-invasive markers and biopsy for the assessment of liver fibrosis in chronic hepatitis B", WORLD JOURNAL OF GASTROENTEROLOGY, WJG PRESS, CN, vol. 13, no. 4, 28 January 2007 (2007-01-28), pages 525-531, XP002596696, ISSN: 1007-9327
- BOURLIERE M ET AL: "Optimized stepwise combination algorithms of non-invasive liver fibrosis scores including Hepascore in hepatitis C virus patients", WORLD JOURNAL OF GASTROENTEROLOGY, WJG PRESS, CN, vol. 28, no. 4, 15 August 2008 (2008-08-15), pages 458-467, XP002596697, ISSN: 1007-9327, DOI: 10.1111/J.1365-2036.2008.03742.X
- HALFON PHILIPPE ET AL: "Comparison of test performance profile for blood tests of liver fibrosis in chronic hepatitis C", JOURNAL OF HEPATOLOGY, ELSEVIER, AMSTERDAM, NL, vol. 46, no. 3, 1 March 2007 (2007-03-01), pages 395-402, XP002596698, ISSN: 0168-8278, DOI: 10.1016/J.JHEP.2006.09.020
- ISAO SAKAIDA ET AL: "Quantitative analysis of liver fibrosis and stellate cell changes in patients with chronic hepatitis C after interferon therapy", THE AMERICAN JOURNAL OF GASTROENTEROLOGY, vol. 94, no. 2, 1 February 1999 (1999-02-01), pages 489-496, XP055195593, ISSN: 0002-9270, DOI: 10.1111/j.1572-0241.1999.884_m.x

**Description**

This invention relates to the field of hepatic diagnosis and more precisely to an in-vitro non-invasive method for quantifying liver lesions, especially due or related to liver impairment, liver steatosis, non-alcoholic fatty liver disease (NAFLD), or non-alcoholic steatohepatitis (NASH). By quantifying in the meaning of this invention is meant determining the amount and/or architecture of hepatic lesions.

FLD (Fatty Liver Disease) describes a wide range of potentially reversible conditions involving the liver, wherein large vacuoles of triglyceride fat accumulate in hepatocytes via the process of steatosis (i.e. the abnormal retention of lipids within a cell).

FLD is commonly associated with alcohol or metabolic syndromes (diabetes, hypertension, dyslipidemia, abetalipoproteinemia, glycogen storage diseases, Weber-Christian disease, Wolman disease, acute fatty liver of pregnancy, lipodystrophy). However, it can also be due to nutritional causes (malnutrition, total parenteral nutrition, severe weight loss, refeeding syndrome, jejuno-ileal bypass, gastric bypass, jejunal diverticulosis with bacterial overgrowth), as well as various drugs and toxins (amiodarone, methotrexate, diltiazem, highly active antiretroviral therapy, glucocorticoids, tamoxifen, environmental hepatotoxins) and other diseases such as inflammatory bowel disease or HIV.

Whether it is AFLD (Alcoholic Fatty Liver Disease) or NAFLD (Non-Alcoholic Fatty Liver Disease), FLD encompasses a morphological spectrum consisting from the mildest type "liver steatosis" (fatty liver), called NAFLD, to the potentially more serious type "steatohepatitis", called NASH, which is associated with liver-damaging inflammation and, sometimes, the formation of fibrous tissue. In fact, steatohepatitis has the inherent propensity to progress towards the development of fibrosis then cirrhosis which can produce progressive, irreversible liver scarring or towards hepatocellular carcinoma (liver cancer).

Because these diseases can be potentially reversed if diagnosed early enough, or at least their consequences limited, it is crucial to be able to provide the medical field with tools that permit such an early, rapid and precise diagnosis.

For a long time, the diagnosis of liver steatosis has usually been accomplished by measuring markers such as g-glutamyltranspeptidase (GGT) and alanine aminotransferase (ALT) while at the same time performing a liver biopsy in order to confirm FLD and determine the grading and staging of the disease. Although biopsies can provide important information regarding the degree of liver damage, in particular the severity of necroinflammatory activity, fibrosis and steatosis, the procedure also presents several limitations, such as sampling error, invasiveness, cost, pain for patients which in turn brings forth a certain reluctance to undergo such a procedure; and finally complications may arise from such procedure, which in some cases can even lead to mortality. Ultrasonography is also used to diagnose liver steatosis. However, this method is subjective as it is based on echo intensity (echogenicity) and special patterns of echoes (texture). As a result, it is not sensitive enough and often inaccurate in patients with advanced fibrosis. Finally, it is generally admitted that around half of all FLD cases are detected by usual blood tests and around half by ultrasonography, resulting in around one quarter of missed diagnosis when both are used.

In recent years, the use of non-invasive biomarkers has gained importance in the field of hepatic diagnosis. Indeed, several tests using non-invasive biomarkers have already been developed and proposed for the diagnosis of fibrosis (see for example WO 2005/116901) . The test relates to a method of diagnosing the presence and/or severity of a hepatic pathology and/or of monitoring the effectiveness of a curative treatment against a hepatic pathology in an individual, by establishing at least one non-invasive diagnostic score, in particular a diagnostic score for portal and septal fibrosis and/or an estimate for the amount of fibrosis (the area of fibrosis) and/or an estimate for the architecture of fibrosis (fractal dimension).

Recently, Poynard et al. (WO 2006/082522) have demonstrated that a single or a panel of biomarkers can be used as an alternative to liver biopsy for the diagnosis of steatosis, whether induced by alcohol, viral hepatitis or NAFLD, the most common causes of steatosis. In particular, this document provides for a new panel of biomarkers known as a SteatoTest (ST) with predictive values for the diagnosis of steatosis due to alcohol, NAFLD and hepatitis C and B. Serum GGT and ALT were considered as the standard biochemical markers. According to the French National Agency for Health (HAS), updated in December 2008, and current international opinion, the performance of this kind of test may be insufficient, especially due to the reference based on a subjective grading of liver steatosis with a poor inter-observer reproducibility. In addition, this grading is a semi-quantitative variable which implies an imprecise and limited reflect of the original pattern.

Watkins et al. (WO 2008/021192) has provided methods for assessing the level of triglycerides in the liver of a subject. Such methods comprise determining the amount of lipid metabolites in a sample collected from a body fluid of the subject and comparing it with a reference value representing the normal level of the lipid metabolites, or correlating the amount with the presence of a liver disorder. The methods are said to be used, for example, for the diagnosis and monitoring of liver disorders such as steatosis, NAFLD and NASH (Non-Alcoholic Steato-Hepatitis). Thus, in order to predict steatosis, Watkins only uses one type of biomarkers (i.e. metabolic lipids) in a random body fluid. However, using this type of biomarkers has several limitations. First, it has been shown that serum triglyceride level was not always an independent marker of liver steatosis. Second, pathological processes are multiple step processes and a targeted strategy for biomarkers can miss one or several steps that are relevant for an accurate diagnosis. Finally, a body fluid is a result of mixing sources from the whole metabolism that can introduces some biases resulting from changes due to other organs than the target organ for diagnosis. Finally, triglyceride content (biochemical measurement) is a different diagnostic target compared to liver steatosis (histological lesion, i.e. abnormal image)

The aim of the present invention is to provide new non-invasive methods using mixed biomarkers from different sources, which circumvent the above-mentioned limitations and are more precise and reliable than the methods cited in the prior art, as shown by their high diagnostic performance. The methods of the invention considerably reduce the need of biopsies, as they catch more information than in the prior art about the lesions evaluated, they ensure reproducibility and performance, while attenuating causes of false results (generally, sources of false results are antagonized in a score including several markers provided that some precautions are included).

The Applicant has now observed that, in the liver, the three lesions, fibrosis, steatosis and inflammation (NASH) are deeply interconnected. For example, the grade of liver steatosis is a predictive factor of NASH in patients with NAFLD; also, significant fibrosis is associated with the development of steatosis and NASH in NAFLD.

The Applicant also observed that, as a hepatic disease is due to the cause, leading to lesions, said lesions inducing symptoms, and ending up into complications, the most reliable information for the patient was the degree of his/her hepatic lesions.

The Applicant hereby shows that the lesions are related: there is a relationship between lesions.
Fibrosis may be quantified by determination of a score (reflecting stage), area of fibrosis (reflecting amount) or fractal dimension (reflecting architecture).
Steatosis may be quantified by determination of a score, area of fibrosis or fractal dimension.
NASH may be evaluated by determination of score(s).

The present invention is defined according to the appended claims. This invention thus relates to an in vitro method for quantifying the lesions of a patient, preferably with NAFLD, comprising addressing a diagnostic target, i.e. quantifying fibrosis, steatosis and/or NASH by

- carrying out a score, an area or a fractal dimension of fibrosis in said patient and/or
- carrying out a score, an area or a fractal dimension of steatosis in said patient and/or
- carrying out a score of steato-hepatitis (NASH) in said patient

through

- measuring at least one, preferably 2,3,4,5,6,7,8,9 marker(s) selected from the group consisting of biomarkers and possibly clinical markers and possibly scores, the biomarkers being selected from the group consisting of glycemia, AST (aspartate aminotransferase), ALT (alanine aminotransferase), AST/ALT, AST.ALT, ferritin, platelets, prothrombin index, hyaluronic acid, haemoglobin, triglycerides, the clinical biomarker being selected from the group consisting of weight, body mass index, sex and age and the scores being selected from the group consisting of score of fibrosis, area of fibrosis, fractal dimension of fibrosis, score of steatosis, area of steatosis, fractal dimension of steatosis;
- and combining said measures in a mathematical function including said markers in order to obtain an end value.

In the last step, combining is obtained by implementing the statistical method involving a mathematical function called binary (or ordinal) logistic regression or multiple linear regression with the following procedure:

- first, the independent variables are tested by univariate analysis;
- second, the independent variables that were significant in univariate analysis are tested in multivariable analysis by binary logistic (or ordinal) regression or multiple linear regression with ascending or descending step by stepwise selection; the selection of variables can be performed in two ways

  ∘ The conventional one where the full sample of patient population is used. This selection procedure could also include score(s) of other lesions than the diagnostic target.
  ∘ The bootstrap method where the mathematical function is applied to different samples with less patients sorted by chance, usually 1000 samples. The selected independent variables are those with an absolute majority occurrence, i.e. at least 500 times among 1000 samples. This method also included only variables without significant collinearity (this means variables that are strongly linked). This procedure did not include score(s) of other lesions than the diagnostic target. Thus, this step provided a minimum number of predictive variables compared to the previous step.

- the logistic regression produces the formula for each score in the form:

$$\texttt{score = a0 + a1 x1 + a2 x2 +}$$

wherein the coefficients ai are constants and the variables xi are the independent variables; this score corresponds to the logit of p where p is the probability of existence of the diagnostic target. This probability p is calculated with the following formula:

$$\texttt{p = exp(}a_0 \texttt{ + } a_1 x_1 \texttt{ + } a_2 x_2 \texttt{ + ...)/(1+exp(}a_0 \texttt{ + } a_1 x_1 \texttt{ + } a_2 x_2 \texttt{ + ...))}$$

or

$$\texttt{p = 1/(1+exp( - }a_0 \texttt{ - } a_1 x_1 \texttt{ - } a_2 x_2 \texttt{ - ...))}$$

wherein the coefficients ai and the variables xi correspond to those of the formula for the score.

We give below the value of the associated coefficients ai (called [beta] in the text below and often in the literature and B in the tables below), and the last two columns give the ai confidence interval, i.e. the confidence interval (called CI in the tables below) of the beta coefficients or corresponding odds-ratio (called exp([beta]) in the tables).

When the diagnostic target is quantitative (discrete mathematical variable) such as the measurement of area of steatosis, the function used is a multiple linear regression with area of steatosis =

$$\texttt{score = a0 + a1 x1 + a2 x2 + ...}$$

In one embodiment, the target is fibrosis, and a score, area and/or fractal dimension of fibrosis is performed, and at least 5, preferably 5,6,7,8 markers, are measured, said markers being selected from the group consisting of glycemia, AST, ALT, ferritin, platelets, prothrombin index, hyaluronic acid, haemoglobin, triglycerides, weight, body mass index, sex and age, preferably glycemia, AST, ALT, ferritin, platelets, weight and age or glycemia, AST, ALT, prothrombin index, weight.

In another embodiment, the target is steatosis and a score, area and/or fractal dimension of steatosis is performed, and at least 5 markers are measured, said markers being selected from the group consisting of glycemia, AST, ALT, AST/ALT, AST.ALT ferritin, platelets, prothrombin index, hyaluronic acid, haemoglobin, triglycerides, weight, body mass index, sex and age, and preferably but not mandatory, a score, area or fractal dimension of fibrosis.

In a further embodiment, the target is steato-hepatitis, and a NASH score is performed.

In another embodiment, the diagnostic target is fibrosis, and

- a score is carried out wherein the biomarkers are selected from the group consisting of weight, age, glycemia, AST, ALT, ferritin and platelets or from the group consisting of weight, age, glycemia, AST, ALT, ferritin and prothrombin index; or
- an area is carried out wherein the biomarkers are selected from the group consisting of hyaluronic acid, glycemia, AST, ALT, platelets, and prothrombin index; or
- a fractal dimension is carried out wherein the biomarkers are selected from the group consisting of weight, hyaluronic acid, glycemia, AST, age, and prothrombin index.

In another embodiment, the diagnostic target is steatosis and

- a score is carried out wherein the biomarkers are selected from the group consisting of glycemia, AST/ALT, triglycerides, haemoglobin, age, and sex and optionally at least one fibrosis score; or from the group consisting of glycemia, AST, triglycerides, AST/ALT, haemoglobin, BMI, and weight and optionally fibrosis scores and/or fractal dimension of fibrosis score; or from the group consisting of BMI, glycemia, and triglycerides, or from the group consisting of BMI, glycemia, triglycerides and ferritin; or
- an area is carried out wherein the biomarkers are selected from the group consisting of glycemia, AST, triglycerides, BMI, weight and optionally at least one fibrosis score; or from the group consisting of BMI, glycemia, triglycerides and ferritin; or
- a fractal dimension is carried out wherein the biomarkers are selected from the group consisting of glycemia, AST, ALT, triglycerides, BMI, weight and optionally at least one fibrosis score; or from the group consisting of BMI, glycemia, triglycerides and ferritin.

In another embodiment, the diagnostic target is NASH, and the biomarkers are selected from the group consisting of AST, ferritin, and AST.ALAT and optionally fibrosis score and/or steatosis score and/or NASH score; or from the group consisting of BMI, AST, and ferritin; or ferritin alone.

**Score#1**

According to a first embodiment, the diagnostic target is significant fibrosis determined by Metavir scoring system implemented by Bedossa et al., and the method of the invention is performed by measuring the level of at least one, preferably 2,3,4,5,6,7, more preferably all markers selected from the group consisting of glycemia, AST, ALT, AST/ALT, ferritin, platelets and measuring the clinical markers weight and age, combining said measures through a mathematical function as described above with the conventional method.

| DIAGNOSTIC TARGET | | INDEPENDENT VARIABLES | SCORE |
|---|---|---|---|
| SIGNIFICANT FIBROSIS | METAVIR F$\geq$ 2 | WEIGHT, AGE, GLYCEMIA, AST, ALT, FERRITIN, PLATELETS | #1A |

Preferably, the coefficients of the mathematical function are as described in the examples.
When selected with the bootstrap method, the variables are unchanged for this score.

| DIAGNOSTIC TARGET | | INDEPENDENT VARIABLES | SCORE |
|---|---|---|---|
| SIGNIFICANT FIBROSIS | METAVIR F$\geq$ 2 | WEIGHT, AGE, GLYCEMIA, AST, ALT, FERRITIN, PLATELETS | #1B |

Preferably, the coefficients of the mathematical function are as described in the examples.

**Score#2**

According to a second embodiment, the diagnostic target is significant fibrosis determined by the NASH-CRN system implemented by Kleiner et al., and the method of the invention is performed by measuring the level of at least one, preferably 2,3,4, more preferably all markers selected from the group consisting of glycemia, AST, ALT, ferritin, prothrombin index and measuring the clinical markers age, weight and combining said measures through a mathematical function as described above with the conventional method.

| DIAGNOSTIC TARGET | | INDEPENDENT VARIABLES | SCORE |
|---|---|---|---|
| SIGNIFICANT FIBROSIS | | | |
| | | | |
| | NASH-CRN F$\geq$ 2 | WEIGHT, AGE, GLYCEMIA, AST, ALT, FERRITIN, PROTHROMBIN INDEX | #2A |

Preferably, the coefficients of the mathematical function are as described in the examples.
When selected with the bootstrap method, the variables are less numerous for this score.

| SIGNIFICANT FIBROSIS | | | |
|---|---|---|---|
| | | | |
| | NASH-CRN F$\geq$ 2 | WEIGHT, GLYCEMIA, AST, ALT, PROTHROMBIN INDEX | #2B |

Preferably, the coefficients of the mathematical function are as described in the examples.

**Score#3**

According to a third embodiment, the diagnostic target is area of fibrosis, and the method of the invention is performed by measuring the level of at least one, preferably 2, 3, 4, 5, 6, more preferably all markers selected from the group consisting of hyaluronic acid, glycemia, AST, ALT, platelets, prothrombin index and combining said measures through a mathematical function as described above.

| DIAGNOSTIC TARGET | INDEPENDENT VARIABLES | SCORE |
|---|---|---|
| AREA OF FIBROSIS | HYALURONIC ACID, GLYCEMIA, AST, ALT, PLATELETS, PROTHROMBIN INDEX | #3A |

Preferably, the coefficients of the mathematical function are as described in the examples.
When selected with the bootstrap method, the variables are unchanged for this score.

| AREA OF FIBROSIS | HYALURONIC ACID, GLYCEMIA, AST, ALT, PLATELETS, PROTHROMBIN INDEX | #3B |
|---|---|---|

Preferably, the coefficients of the mathematical function are as described in the examples.

**Score#4**

According to a fourth embodiment, the diagnostic target is fractal dimension of fibrosis, and the method of the invention is performed by measuring the level of at least one, preferably 2, 3, 4, 5, more preferably all markers selected from the group consisting of hyaluronic acid, glycemia, AST, prothrombin index and the clinical marker weight, age, combining said levels in a mathematical function, obtained as described above.

| DIAGNOSTIC TARGET | INDEPENDENT VARIABLES | SCORE |
|---|---|---|
| FRACTAL DIMENSION OF FIBROSIS | HYALURONIC ACID, GLYCEMIA, AST, AGE, PROTHROMBIN INDEX | #4A |

Preferably, the coefficients of the mathematical function are as described in the examples.
When selected with the bootstrap method, the variables are the following:

| FRACTAL DIMENSION OF FIBROSIS | HYALURONIC ACID, GLYCEMIA, AST/ALT, WEIGHT, PLATELETS | #4B |
|---|---|---|

Preferably, the coefficients of the mathematical function are as described in the examples.

**Score#5**

According to a fifth embodiment, the diagnostic target is significant steatosis determined according to a threshold of area of steatosis ($\geq 3\%$), and the method of the invention is performed by measuring the level of at least one, preferably 2, 3, 4, 5,6, more preferably all markers selected from the group consisting of glycemia, AST/ALT, triglycerides, haemoglobin, age and sex, and optionally a score as obtained from the first embodiment (score #1A) or the second embodiment (score#2A) of the present invention, as described above, and combining said levels in a mathematical function, obtained as described above.

| DIAGNOSTIC TARGET | | INDEPENDENT VARIABLES | SCORE |
|---|---|---|---|
| Significant steatosis | rAOS $\geq 3\%$ | GLYCEMIA, AST/ALT, TRIGLYCERIDES, HAEMOGLOBIN, AGE, SEX + optionally SCORE 1A AND/OR SCORE 2A | #5A |

Preferably, the coefficients of the mathematical function are as described in the examples.

When selected with the bootstrap method, the variables are the following:

| SIGNIFICANT STEATOSIS | rAOS $\geq$ 3% | BMI, GLYCEMIA, TRIGLYCERIDES | #5B |
|---|---|---|---|

Preferably, the coefficients of the mathematical function are as described in the examples.

### Score #6

In the sixth embodiment, the diagnostic target is significant steatosis determined by NASH-CRN GRADE $\geq$ 1, and the method of the invention is performed by measuring the level of the biomarkers selected from glycemia, AST, triglycerides, AST/ALT, haemoglobin, the clinical markers are selected from the group consisting of BMI and weight, and optionally, one or more of the score 1, 2 and/or 4 and combining said levels in a mathematical function, obtained as described above.

| DIAGNOSTIC TARGET | | INDEPENDENT VARIABLES | SCORE |
|---|---|---|---|
| SIGNIFICANT STEATOSIS | NASH-CRN GRADE $\geq$1 | GLYCEMIA, AST, TRIGLYCERIDES, AST/ALT, HAEMOGLOBIN, BMI, WEIGHT + SCORES 1A, 2A AND 4A | #6A |

Preferably, the coefficients of the mathematical function are as described in the examples.
When selected with the bootstrap method, the variables are the following:

| SIGNIFICANT STEATOSIS | NASH-CRN GRADE $\geq$1 | BMI, GLYCEMIA, TRIGLYCERIDES, FERRITIN | #6B |
|---|---|---|---|

Preferably, the coefficients of the mathematical function are as described in the examples.

### Score #7

In the seventh embodiment, the diagnostic target is area of steatosis, and the method of the invention is performed by measuring the level of at least one, preferably 2, 3, 4, 5, more preferably all markers selected from the group consisting of glycemia, AST, triglycerides, ferritin, BMI and weight, and optionally, one or more of the scores 1 and/or 2, combining said levels in a mathematical function, obtained as described above.

| DIAGNOSTIC TARGET | INDEPENDENT VARIABLES | SCORE |
|---|---|---|
| AREA OF STEATOSIS | GLYCEMIA, AST, TRIGLYCERIDES, BMI, WEIGHT + OPTIONNALY SCORES 1 AND 2 | #7A |

Preferably, the coefficients of the mathematical function are as described in the examples.
When selected with the bootstrap method, the variables are the following:

| AREA OF STEATOSIS (LOG) | BMI, GLYCEMIA, TRIGLYCERIDES, FERRITIN | #7B |
|---|---|---|

Preferably, the coefficients of the mathematical function are as described in the examples.

### Score#8

In the eighth embodiment, the diagnostic target is fractal dimension of steatosis, and the method of the invention is performed by measuring the level of at least one, preferably 2, 3, 4, 5, more preferably all markers selected from the group consisting of glycemia, AST, ALT/ALAT, AST.ALT, ferritin, triglycerides, BMI, weight, and optionally scores 1A and 2A and combining said levels in a mathematical function, obtained as defined in embodiment 1 and 2 here above.

| FRACTAL DIMENSION OF STEATOSIS | GLYCEMIA, AST, ALT/ALAT, AST.ALT, TRIGLYCERIDES, BMI, WEIGHT + SCORES 1A AND 2A | #8 |
|---|---|---|

Preferably, the coefficients of the mathematical function are as described in the examples.

When selected with the bootstrap method, the variables are the following:

| FRACTAL DIMENSION OF STEATOSIS | BMI, GLYCEMIA, TRIGLYCERIDES, FERRITIN | #8B |
|---|---|---|

Preferably, the coefficients of the mathematical function are as described in the examples.

**Score #9**

In the ninth embodiment, the diagnostic target is NASH according to three definitions based on NASH activity score (NAS) by Kleiner et al., and the method of the invention is performed by measuring the level of at least one, preferably 2, 3, 4, more preferably all markers selected from the group consisting of AST, ferritin, AST.ALT, BMI, and optionally scores 2A and/or 5A and/or 8A and combining said levels in a mathematical function, obtained as described above.

| NASH | AST, FERRITIN, AST.ALAT + OPTIONALLY SCORES 2A, 5A AND/OR 8A | #9A |
|---|---|---|

Preferably, the coefficients of the mathematical function are as described in the examples.

When selected with the bootstrap method, the variables are the following:

| NAS $\geq$ 3 | BMI, AST, FERRITIN | #9B |
|---|---|---|
| NAS $\geq$ 5 | FERRITIN | #10B |
| NAS 0-2 / 3-4 / 5-7 | BMI, AST, FERRITIN | #11B |

Preferably, the coefficients of the mathematical function are as described in the examples.

In the context of the present invention, the following definitions are to be applied:

- Fibrosis is defined as a pathological lesion made of scarring tissue including fibrillar proteins or glycoproteins (collagens, proteoglycans...) .
- Fibrosis Score is defined as a combination of markers (usually linked by a mathematical function) for which the diagnostic target is fibrosis.
- Steatosis is defined as an accumulation of lipids, usually triglycerides, within vacuoles in cells. It mainly concerns liver and muscle in metabolic syndrome.
- Area of steatosis (AOS) is defined as the liver surface occupied by the vacuoles of steatosis as seen on a liver specimen observed in two dimensions.
- Significant area of steatosis (SAOS) is defined as a threshold clinically and statistically significant of AOS, in the setting of NAFLD, e.g. more than or equal to 3% of liver surface.
- Kleiner grading/staging: this is a pathological classification devoted to NAFLD based on a morphological description in different classes either for steatosis (conventionally referred to as grading) or fibrosis (conventionally referred to as staging). This semi-quantitative (ordinal in statistics) system is the most recent and conventional histological classification. This system is also known as the NASH Clinical Research Network (NASH-CRN) system.
- Metavir grading/staging: this is a pathological classification devoted to chronic viral hepatitis based on a morphological description in different classes either for necro-inflammatory activity (conventionally referred to as grading) or fibrosis (conventionally referred to as staging). This semi-quantitative (ordinal in statistics) system is the most used histological classification in viral hepatitis. It is also used in other causes of chronic liver diseases such as alcohol and NAFLD.
- Level: the quantity of a particular biomarker measured in the blood or plasma of a patient, usually expressed as a concentration.

**Target is steatosis**

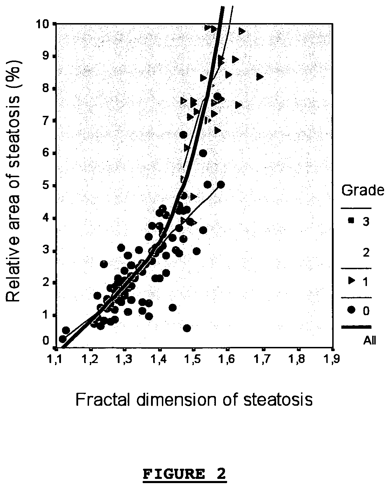

The Applicant especially focused on the target "steatosis," and found that the inclusion of fibrosis score in an *in vitro* method for diagnosing a liver condition involving steatosis, might be of high interest because of the interaction between fibrosis and steatosis (see figure 2 showing that the relationship between AOS and fibrosis stages explains that the inclusion of non-invasive measurement of fibrosis stage increases the accuracy of non-invasive measurement of AOS)

Thus, according to an embodiment, a method of the invention comprises measuring the level of at least one, preferably 2, 3, 4, 5, 6, 7, 8 biological marker (s) and at least one, preferably two, clinical marker (s) and optionally, but preferably, a Fibrosis Score, and combining said levels, measures and score in a suitable mathematical function.

According to a first embodiment, the at least one biological marker is selected from the group consisting of glycemia (GLY), aspartate aminotransferase (AST), alanine aminotransferase (ALT), AST/ALT, hyaluronic acid (HA), haemoglobin (Hb) and triglycerides, preferably glycemia (GLY), aspartate aminotransferase (AST), alanine aminotransferase (ALT), AST/ALT, hyaluronic acid (HA), haemoglobin (Hb) and triglycerides; the level of these blood markers may be easily dosed with methods already known in the art; preferably two clinical markers are selected from the group consisting of age, sex, BMI, weight; preferably, a score is performed.

According to a second embodiment, the Fibrosis Score of the present invention is determined through the method described in WO2005/116901, herein incorporated by reference.

According to a further embodiment, the Fibrosis Score is determined by measuring in a sample of said patient and combining in a logistic regression function at least three markers selected in the group consisting of $\alpha$-2 macroglobulin (A2M), hyaluronic acid (HA or hyaluronate), apoliprotein A1 (ApoA1), N-terminal propeptide of type III procollagen (P3P), gamma-glutamyltranspeptidase (GGT), bilirubin, gamma-globulins (GLB), platelets (PLT), prothrombin level (TP), aspartate aminotransferase (AST), alanine aminotransferase (ALT), urea, sodium (NA), glycemia (GLY), triglycerides (TG), albumin (ALB), alkaline phosphatases (PAL), YKL-40 (human cartilage glycoprotein 39), tissue inhibitor of matrix metalloproteinase 1 (TIMP-1), matrix metalloproteinase 2 (MMP-2), ferritin.

According to another embodiment, the Fibrosis Score is measured by combining the levels of at least three biological markers selected from the group consisting of glycemia (GLY), aspartate aminotransferase (AST), alanine aminotransferase (ALT), ferritin, hyaluronic acid (HA), triglycerides (TG), prothrombin index (PI) gamma-globulins (GLB), platelets (PLT), weight, age and sex.

According to yet another embodiment, at least 2, preferably at least 3, more preferably at least 4, even more preferably at least 5 biological markers, and even most preferably at least 6 biological markers and optionally Fibrosis Score are measured and combined in the method of the present invention.

According to another embodiment, the method according to the invention further comprises measuring at least one clinical marker. Preferably the clinical marker is selected from the group consisting of the age (AGE), the body mass index (BMI), the body weight, the hip perimeter, the abdominal perimeter and a ratio thereof, such as for example hip perimeter/abdominal perimeter; more preferably two clinical markers are measured.

According to yet another embodiment, the method according to the invention comprises measuring at least three biomarkers selected from the group consisting of glycemia (GLY), aspartate aminotransferase (AST), alanine aminotransferase (ALT), hyaluronic acid (HA), haemoglobin (Hb) and triglycerides, and at least one, preferably two clinical markers selected from the group consisting of the age (AGE), the body mass index (BMI), the body weight, the hip perimeter, the abdominal perimeter and a ratio thereof, such as for example hip perimeter/abdominal perimeter and a Fibrosis score and combining said levels of biological markers, measured Fibrosis Score and measured clinical markers, through a suitable mathematical function, preferably a logistic function or a multiple regression function.

**Steatosis by MRI**

Another disclosure is a method for quantifying the area of liver steatosis (AOS) in a patient, comprising performing a multi-echo gradient-echo MRI called MFGRE on whole or part of the liver of the patient, measuring the liver fat content on the resulting MRI signal and comparing said liver fat content on the resulting MRI signal to the area of lipid vacuoles of the reference image.

(MFGRE) is a new method developed by the applicant and based on a multi-echo prototype MRI sequence. The principle of the sequence is an echo gradient in-phase and out-of-phase but with the acquisition of 16 echoes, allowing the precise calculation of signal decay parameters: water signal (W), fat (F) signal, local field in homogeneity (F) and noise (N). Fat fraction is calculated using the following formula:

$$\frac{F}{W+F} \times 100 = \% F_{MGRE}$$

With this method, MFGRE is used as a non-invasive marker of steatosis either *per se* or with equivalence in AOS by using a linear regression score. This marker can be used as part of a test in the same way as a blood or clinical marker.

Another disclosure is a method for quantifying an area of liver steatosis (AOS) in a patient, comprising:

> a) measuring the fat content on a MRI signal of the liver of a patient;
> b) measuring the level of at least one biological marker and/or Fibrosis Score, and/or at least one, preferably two, clinical marker(s);
> c) combining the measured fat content index obtained in step (a) and the measured levels, score and/or clinical markers obtained in step (b) in a suitable mathematical function.

According to yet another embodiment, the method for quantifying an area of liver steatosis (AOS) measures at least 2, preferably at least 3, more preferably at least 4 and even more preferably at least 5 biological markers and most preferably at least 6 biological markers and optionally Fibrosis Score in step (b).

Another disclosure is a method for diagnosing the presence and/or the severity of a liver steatosis or fatty liver disease in a patient, comprising implementing a method of measuring the area of steatosis as described in the present invention. The method of the invention is of high interest to diagnose and quantify main liver lesions, especially NAFLD lesions.

Other objects, advantages and features of the present invention will become more apparent upon reading of the following non restrictive description of preferred embodiments thereof, given by way of examples with reference to the accompanying figures.

### Brief description of the figures

> **Figure 1** Correlation between morphometric characteristics of liver fibrosis or steatosis and corresponding blood tests as a function of fibrosis stages or steatosis grades. Panel a: area of fibrosis, panel b: fractal dimension of fibrosis. Panel c: area of steatosis, panel d: fractal dimension of steatosis. The lines depict the linear regression.

> **Figure 2** Relationship between the area and fractal dimension of steatosis as a function of steatosis grades. The lines depict the LOWESS regression curves. The Y axis was truncated to show more details.

> **Figure 3** Relationship between blood tests for NAS (panel a) or NASH (panel b) (Y axis) and NAS (X axis). Box plots (median, interquartile range and extremes).

> **Figure 4** Relationship between area and fractal dimension as a function of fibrosis stages or steatosis grades. Left panels (a and c): morphometric results. Right panels (b and d) : blood tests. Upper panels (a and b) : fibrosis; bottom panels (c and d) : steatosis. The lines depict the LOWESS regression curves.

> **Figure 5** Relationship between area or fractal dimension of fibrosis or steatosis (Y axis) as a function of fibrosis stages (X axis). Left panels (a and c): morphometric results. Right panels (b and d): blood tests. Upper panels (a and b): area; bottom panels (c and d): fractal dimension. Box plots (median, interquartile range and extremes).

> **Figure 6** depicts the correlation between the AOS (X axis) and the MFGRE sequence by MRI (Y axis) in 23 patients: Rs = 0.77 and Ric = 0.85.

### EXAMPLES

### PATIENTS AND METHODS

### Patients

Two tertiary centers, Angers and Rennes (France), prospectively recruited 245 patients with NAFLD between 2001 and 2006. Inclusion and exclusion criteria are described elsewhere (Cales P et al, J Hepatol 2009, 50:165-173). Nineteen patients were not included because their liver specimens were not available for rereading; thus, the core population included 226 patients. Additional 22 liver biopsies were unsuitable for image analysis.

**Clinical data and blood tests**

Fasting blood samples were taken at inclusion (date of liver biopsy $\pm7$ days). The usual clinical and blood variables were included, especially fasting glucose at inclusion, as were the following fibrosis markers: hyaluronic acid, $\alpha$2-macroglobulin, apolipoprotein A1, prothrombin index, platelets, aspartate and alanine aminotransferases (AST, ALT), $\gamma$-glutamyltranspeptidase, total bilirubin and urea. Methods and reagents were previously described (Cales P et al, Clin Biochem 2008, 41:10-18). One blood fibrosis score specific for NAFLD was calculated according to published score (Cales P et al, J Hepatol 2009, 50:165-173). The inter-laboratory reproducibility of blood markers was evaluated as excellent in a previous study (Cales P et al, Clin Biochem 2008, 41:10-18).

**Liver biopsy**

A percutaneous liver biopsy was performed generally within one week (maximum 3 months) of blood sampling. Specimen lengths were measured before paraffin-embedding. Then, 5 $\mu$m-thick sections were stained with hematoxylin-eosin-saffron or 0.1 % picrosirius red solution and used for both optical and image analyses.

*Optical analysis*

Biopsy specimens were centrally re-examined by two liver pathologists; discordant cases were reviewed by a third pathologist to reach consensus. Steatosis, lobular inflammation and hepatocyte ballooning were separately graded according to the NASH Clinical Research Network (NASH-CRN) system (Kleiner DE et al, Hepatology 2005, 41:1313-1321). The addition of these 3 grades provided the NAFLD activity score (NAS). The diagnosis of NASH was evaluated according to three definitions based on NAS: $\geq 3$, $\geq 5$ and 0-2 / 3-4 / 5-7, respectively no, possible/borderline, definite NASH (Kleiner DE et al, Hepatology 2005, 41:1313-1321). Fibrosis was graded into 5 stages, called here F0 to F4, according to NASH-CRN staging specifically developed for NAFLD (Kleiner DE et al, Hepatology 2005, 41:1313-1321). The Metavir staging (Hepatology 1994, 20: 15-20) developed for viral hepatitis, but also used in alcoholic chronic liver diseases and in NAFLD, was also evaluated to stage porto-septal fibrosis in acinar zone 1.

*Image analysis*

*Image acquisition* - We used an Aperio digital slide scanner (Scanscope® CS System, Aperio Technologies, Vista CA 92081, USA) image processor that provided high quality 30,000 x 30,000 pixel images at a resolution of 0.5 $\mu$m/pixel (magnification x20). A binary image (white and black) was obtained via an automatic thresholding technique using an algorithm developed in our laboratory. The entire specimen area was analyzed. Liver specimen artifacts (folds, dust) were manually removed.

*Algorithm for steatosis* - Some aspects of the algorithm have been previously described (Roullier V et al., Conf Proc IEEE Eng Med Biol Soc 2007; 2007:5575-5578) and techniques providing images and measurements of AOS have been recently developed (Boursier J et al., J Hepatol 2009, 50:S357).

*Calculated data* - AOF was measured on the complete liver sections, as previously described (Pilette C et al., J Hepatol 1998, 28:439-446) but with several improvements in image acquisition as described above. AOS (%) was calculated as the ratio: area of steatosis vesicles / complete liver surface, and relative AOS (rAOS, %) as the ratio: area of steatosis / non-fibrous liver area (i.e. complete liver surface minus AOF). Finally, the box counting method was used to evaluate the Kolmogorov FD of steatosis and fibrosis as detailed elsewhere (Moal F et al., Hepatology 2002, 36:840-849).

**Statistical analysis**

Quantitative variables were expressed as mean $\pm$ SD, unless otherwise specified. The Lowess regression by weighted least squares was used to determine the *average* trend of relationships between variables (Borkowf CB et al. Stat Med 2003, 22:1477-1493). Independent predictors were determined by the bootstrapping method. This method was also determined to calculate the optimism bias.

Diagnostic performance was expressed by the area under the receiver operating characteristic (AUROC) or diagnostic accuracy. Diagnostic cut-offs were determined according to maximum Youden index and diagnostic accuracy. Stepwise multiple linear regression and binary logistic regression were used to determine independent variables. In the linear regression, all categorical variables were dichotomized and the performance of each model was expressed by the adjusted $R^2$ ($aR^2$). Statistics software programs were SPSS version 11.5.1 (SPSS Inc., Chicago, IL, USA) and SAS 9.1 (SAS Institute Inc., Cary, NC, USA).

**RESULTS**

**General characteristics of NAFLD patients**

They are detailed in **table 1.**

**Table 1.** Characteristics of 226 patients with NAFLD included.

| **Variable** | **Value** (% or mean $\pm$) |
|---|---|
| **Demographic:** | |
| Sex (% male) | 75.2 |
| Age (years) | 50.9 $\pm$ 10.8 |
| Body weight (kg) | 82.9 $\pm$ 15.8 |
| BMI (kg/m$^2$) | 28.7 $\pm$ 4.9 |
| **Liver specimen:** | |
| Size[a] (mm) by pathologist | 30.5 $\pm$ 11.9 |
| Size[b] (mm) by computer | 23.4 $\pm$ 23.6 |
| Area[b] (mm$^2$) | 11.7 $\pm$ 11.8 |
| **Fibrosis:** | |
| Fibrosis stage by Metavir (% | 44.2 / 29.2 / 9.3 / |
| Fibrosis stage by NASH-CRN (% | 26.1 / 29.7 / 21.6 / |
| Area of fibrosis (%) | 6.4 $\pm$ 5.4 |
| Fractal dimension | 1.14 $\pm$ 0.20 |
| **Steatosis:** | |
| Steatosis grade (% grade 0/1/2/3) | 45.3 / 21.5 / 21.1 / |
| Area of steatosis (%) | 10.3 $\pm$ 9.7[c] |
| Fractal dimension | 1.52 $\pm$ 0.17 |
| NAFLD activity score: 0-2, 3-4, 5-7 | 53.2 / 30.6 / 16.2 |
| NASH (%) | 46.8 |
| [a] Before paraffin embedding; [b] By computerized analysis of stained digital images; [c] range: 0.2-42.5%; [d] NAS: interpretation: no, possible/borderline, definite NASH. | |

**Diagnosis of fibrosis**

***Significant fibrosis***

*Diagnostic performance* - The prevalence of significant fibrosis was significantly higher with NASH-CRN F$\geq$ 2: 43.9% than with Metavir F$\geq$ 2: 27.6% (p<10$^{-3}$ by McNemar test). We have compared a previous blood test targeted to Metavir F$\geq$ 2, called FibroMeter (FM) and a new model of blood test targeted to NASH-CRN F$\geq$ 2 **(table 2)**. Tables 2 and 3 show very good performance (high AUROC) for the scores of the invention.

**Table 2:** Multivariate analyses showing variables independently associated with different diagnostic targets. Diagnostic performance was expressed by either AUROC for score obtained by binary logistic regression or $_aR^2$ for score obtained by multiple linear regression. In these models, the prediction was the main objective so that conditions of independent variables were less stringent than in an explanatory model.

| Diagnostic target | Independent variables | Score | Performance |
|---|---|---|---|
| Fibrosis: | | | |

(continued)

| Diagnostic target | | Independent variables | Score | Performance |
|---|---|---|---|---|
| Significant fibrosis | Metavir F≥ 2 | Weight, age, glycemia, AST, ALT, ferritin, platelets | #1A | AUROC=0.940 |
| | | | | |
| | NASH-CRN F≥ 2 | Weight, age, glycemia, AST, ALT, ferritin, prothrombin index | #2A | AUROC=0.884 |
| Area of fibrosis | | Hyaluronic acid, glycemia, AST, ALT, platelets, prothrombin index | #3A | aR2=0.520 |
| Fractal dimension of fibrosis | | Hyaluronic acid, glycemia, AST, age, prothrombin index | #4A | aR2=0.524 |
| Steatosis: | | | | |
| Significant steatosis | rAOS ≥ 3% | Glycemia, AST/ALT, triglycerides, haemoglobin, age, sex + scores 1 and 2 | #5A | AUROC=0.847 |
| | | | | |
| | NASH-CRN grade ≥ 1 | Glycemia, AST, triglycerides, AST/ALT, haemoglobin, BMI, weight + scores 1, 2 and 4 | #6A | AUROC=0.848 |
| Area of steatosis | | Glycemia, AST, triglycerides, BMI, weight + scores 1 and | #7A | aR2=0.365 |
| Fractal dimension of steatosis | | Glycemia, AST, ALT, triglycerides, BMI, | #8A | aR2=0.346 |
| | | weight + scores 1 and 2 | | |
| NASH: | | | | |
| NASH | | AST, ferritin, AST.ALAT + scores 2, 5 and 8 | #9A | AUROC=0.895 |
| AST: aspartate aminotransferase ALT: alanine aminotransferase BMI: body mass index | | | | |

**Table 2bis:** Multivariate analyses showing variables independently associated with different diagnostic targets. Diagnostic performance was expressed by either AUROC for score obtained by binary logistic regression or $_aR^2$ for score obtained by multiple linear regression. In these models, the prediction was the main objective so that conditions of independent variables were less stringent than in an explanatory model.

| Score | Diagnostic target | Variables | β coeff | Exp (β) | 95% CI inf Exp (β) | 95% CI sup Exp (β) |
|---|---|---|---|---|---|---|
| **1a** | **Significant fibrosis Metavir F≥ 2** | | | | | |
| N=225 | | Intercept | -11.044 | - | - | - |
| | | Age | 0.071 | 1.074 | 1.021 | 1.130 |
| | | Weight | 0.047 | 1.048 | 1.016 | 1.082 |
| | AUROC = 0.941 | Glycemia | 0.429 | 1.536 | 1.120 | 2.106 |
| | (0.927) | AST | 0.066 | 1.069 | 1.041 | 1.097 |
| | | ALT | -0.024 | 0.976 | 0.960 | 0.993 |
| | | Ferritin | 0.0009 | 1.001 | 1.000 | 1.002 |

(continued)

| Score | Diagnostic target | Variables | β coeff | Exp (β) | 95% CI inf Exp (β) | 95% CI sup Exp (β) |
|-------|-------------------|-----------|---------|---------|--------------------|--------------------|
| | | Platelets | -0.0103 | 0.990 | 0.982 | 0.998 |
| **2a** | **Significant fibrosis NASH-CRN F≥ 2** | | **β coeff** | **Exp (β)** | **95% CI inf Exp (β)** | **95% CI sup Exp (β)** |
| N=221 | | Intercept | -6.269 | - | - | - |
| | | Age | 0.033 | 1.034 | 0.996 | 1.072 |
| | | Weight | 0.053 | 1.055 | 1.024 | 1.086 |
| | AUROC = 0.884 | Glycemia | 0.388 | 1.474 | 1.013 | 2.146 |
| | (0.867) | AST | 0.059 | 1.060 | 1.033 | 1.088 |
| | | ALT | -0.014 | 0.986 | 0.973 | 0.998 |
| | | Ferritin | 0.001 | 1.001 | 1.000 | 1.002 |
| | | Prothrombin index | -0.046 | 0.955 | 0.916 | 0.996 |
| **3a** | **Area of fibrosis** | | **β coeff** | | **95% CI inf β** | **95% CI sup β** |
| N=202 | | Intercept | 11.597 | | **5.724** | **17.470** |
| | | Glycemia | 0.441 | | 0.161 | 0.721 |
| | $_aR^2$ = 0.520 | AST | 0.049 | | 0.023 | 0.076 |
| | (0.503) | ALT | -0.021 | | -0.037 | -0.005 |
| | | Platelets | -0.008 | | -0.016 | 0.001 |
| | | Prothrombin index | -0.089 | | -0.151 | -0.027 |
| | | Hyaluronic acid | 0.021 | | 0.015 | 0.027 |
| **4a** | **Fractal dimension of fibrosis** | | **β coeff** | | **95% CI inf β** | **95% CI sup β** |
| N=202 | | Intercept | 0.920 | | 0.636 | 1.203 |
| | | Age | 0.003 | | 0.001 | 0.005 |
| | $_aR^2$ = 0.507 | Weight | 0.002 | | 0.0003 | 0.003 |
| | (0.495) | Glycemia | 0.025 | | 0.014 | 0.035 |
| | | AST | 0.001 | | 0.0007 | 0.002 |
| | | Prothrombin index | -0.004 | | -0.006 | -0.001 |
| | | Hyaluronic acid | 0.001 | | 0.0003 | 0.0008 |
| **5a** | **Significant steatosis (rAOS ≥3%)** | | **β coeff** | **Exp (β)** | **95% CI inf Exp (β)** | **95% CI sup Exp (β)** |
| N=151 | | Intercept | -5.090 | - | - | - |
| | | Age | -0.065 | 0.937 | 0.880 | 0.998 |

(continued)

| Score | Diagnostic target | Variables | β coeff | Exp (β) | 95% CI inf Exp (β) | 95% CI sup Exp (β) |
|---|---|---|---|---|---|---|
| | | Sex (M:0, F:1) | 1.422 | 4.145 | 0.905 | 18.971 |
| | AUROC = 0.866 | Triglyceride s | 0.956 | 2.602 | 1.203 | 5.624 |
| | | Glycemia | 0.479 | 1.614 | 0.974 | 2.675 |
| | | AST/ALT | -2.588 | 0.075 | 0.010 | 0.559 |
| | | Haemoglobin | 0.238 | 1.269 | 0.765 | 2.105 |
| | | Score 1a | -8.416 | <0.001 | <0.001 | 0.071 |
| | | Score 2a | 13.450 | >999.9 9 | >999.9 9 | >999.9 9 |
| 6a | Significant steatosis NASH-CRN grade≥1 | | β coeff | Exp (β) | 95% CI inf Exp (β) | 95% CI sup Exp (β) |
| N=162 | | Intercept | -2.5933 | - | - | - |
| | | Weight | -0.1066 | 0.899 | 0.846 | 0.956 |
| | | BMI | 0.3864 | 1.472 | 1.189 | 1.822 |
| | AUROC = 0.898 | Glycemia | 1.2142 | 3.368 | 1.738 | 6.525 |
| | | Triglycerides | 0.8002 | 2.226 | 1.141 | 4.344 |
| | | AST | 0.0324 | 1.033 | 1.007 | 1.060 |
| | | AST/ALT | -2.4952 | 0.082 | 0.008 | 0.803 |
| | | Haemoglobin | 0.3510 | 1.421 | 0.899 | 2.246 |
| | | Score 1a | -9.6939 | <0.001 | <0.001 | 0.010 |
| | | Score 2a | 14.8116 | >999.9 9 | >999.9 9 | >999.9 9 |
| | | Score 4a | -14.2847 | <0.001 | <0.001 | 0.674 |
| 7a | Area of steatosis (log) | | β coeff | | 95% CI inf β | 95% CI sup β |
| N=151 | | Intercept | -2.28009 | | - 3.6422 6 | - 0.9179 3 |
| | | Weight | -0.01128 | | - 0.0299 0 | 0.0073 5 |
| | | BMI | 0.10169 | | 0.0383 8 | 0.1650 0 |
| | $_aR^2 = 0.310$ | Glycemia | 0.18799 | | 0.0592 0 | 0.3167 9 |
| | | Triglycerides | 0.20372 | | 0.0412 6 | 0.3661 7 |
| | | AST | 0.00922 | | 0.0022 2 | 0.0162 1 |
| | | Score 1a | -3.24488 | | - 4.5588 1 | - 1.9309 5 |
| | | Score 2a | 2.66894 | | 1.3480 7 | 3.9898 1 |
| 8a | Fractal dimension of steatosis | | β coeff | | 95% CI inf β | 95% CI sup β |
| N=151 | | Intercept | 0.95361 | | 0.7095 6 | 1.1976 6 |
| | | Weight | -0.00238 | | - 0.0052 9 | 0.0005 4241 |

(continued)

| Score | Diagnostic target | Variables | β coeff | Exp (β) | 95% CI inf Exp (β) | 95% CI sup Exp (β) |
|---|---|---|---|---|---|---|
| | | BMI | 0.01580 | | 0.0059 2 | 0.0256 8 |
| | | Glycemia | 0.02984 | | 0.0093 2 | 0.0503 7 |
| | $_aR^2 = 0.350$ | Triglycerides | 0.03147 | | 0.0063 7 | 0.0565 6 |
| | | AST | 0.00438 | | 0.0014 4 | 0.0073 3 |
| | | AST/ALT | -0.18234 | | - 0.3106 0 | - 0.0540 7 |
| | | AST.ALT | -0.00002 | | - 0.0000 3124 | - 0.0000 0319 |
| | | Score 1a | -0.40669 | | - 0.6132 3 | - 0.2001 6 |
| | | Score 2a | 0.43128 | | 0.2191 7 | 0.6433 8 |
| 9a | NASH (NAS≥3) | | β coeff | Exp (β) | 95% CI inf Exp (β) | 95% CI sup Exp (β) |
| N=162 | | Intercept | -20.2665 | - | - | - |
| | | AST | 0.1253 | 1.133 | 1.048 | 1.226 |
| | | AST.ALT | -0.00042 | 1.000 | 0.999 | 1.000 |
| | AUROC = 0.899 | Ferritin | 0.00275 | 1.003 | 1.001 | 1.005 |
| | | Score 2a | -4.3346 | 0.013 | <0.001 | 0.285 |
| | | Score 5a | 4.0033 | 54.779 | 1.932 | >999.9 9 |
| | | Score 8a | 9.4028 | >999.9 9 | 3.123 | >999.9 9 |
| AST: aspartate aminotransferase ALT: alanine aminotransferase BMI: body mass index | | | | | | |

**Table 3:** Multivariate analyses showing variables independently associated with different diagnostic targets. Diagnostic performance was expressed by either AUROC for score obtained by binary/ordinal logistic regression or $_aR^2$ for score obtained by multiple linear regression. Figures into brackets denote AUROC corrected with optimism bias. In these models, the explanation was the main objective so that conditions of independent variables were more stringent than in a predictive model. Variables were selected by the stepwise bootstrap method.

| Diagnostic target | | Independent variables | Score | Performance |
|---|---|---|---|---|
| Fibrosis: | | | | |
| Significant fibrosis | Metavir F≥ 2 | Weight, age, glycemia, AST, ALT, ferritin, platelets a | #1B | AUROC=0.932 (0.918) |
| | | | | |
| | NASH-CRN F≥ 2 | Weight, glycemia, AST, ALT, prothrombin index | #2B | AUROC=0.866 (0.856) |
| Area of fibrosis | | Hyaluronic acid, glycemia, AST, ALT, platelets, prothrombin index | #3B | aR2=0.530 (0.518) |
| Fractal dimension of fibrosis | | Hyaluronic acid, glycemia, AST/ALT, weight, platelets | #4B | aR2=0.529 (0.517) |

(continued)

| Diagnostic target | | Independent variables | Score | Performance |
|---|---|---|---|---|
| Steatosis: | | | | |
| Significant steatosis | rAOS ≥ 3% | BMI, glycemia, triglycerides | #5B | AUROC=0.797 (0.782) |
| | | | | |
| | NASH-CRN grade ≥ 1 | BMI, glycemia, triglycerides, ferritin | #6B | AUROC=0.831 (0.815) |
| Area of steatosis (log) | | BMI, glycemia, triglycerides, ferritin | #7B | aR2=0.213 (0.198) |
| Fractal dimension of steatosis | | BMI, glycemia, triglycerides, ferritin | #8B | aR2=0.247 (0.230) |
| NASH b: | | | | |
| NAS ≥ 3 | | BMI, AST, ferritin | #9B | AUROC=0.846 (0.836) |
| NAS ≥ 5 | | Ferritin | #10B | AUROC=0.709 (0.708) |
| NAS 0-2 / 3-4 / 5-7 | | BMI, AST, ferritin | #11B | AUROC=0.815 |
| AST: aspartate aminotransferase<br>ALT: alanine aminotransferase<br>BMI: body mass index<br>a The original model determined by conventional binary logistic regression<br>b According to 3 definitions based on NAS | | | | |

**Table 3bis:** Multivariate analyses showing variables independently associated with different diagnostic targets. Diagnostic performance was expressed by either AUROC for score obtained by binary/ordinal logistic regression or $_aR^2$ for score obtained by multiple linear regression. Figures into brackets denote AUROC corrected from optimism bias. In these models, the explanation was the main objective so that condition of non-collinearity between independent variables was more stringent than in a predictive model. Variables were selected by a bootstrap method (stepwise regression analysis on 1,000 bootstrap samples).

| Score | Diagnostic target | Variables | β coeff | Exp (β) | 95% CI inf | 95% CI sup |
|---|---|---|---|---|---|---|
| **1b** | **Significant fibrosis Metavir F≥ 2** | | | | **Exp (β)** | **Exp (β)** |
| | | Intercept | -6.9269 | - | - | - |
| | | Age | 0.0671 | 1.069 | 1.016 | 1.126 |
| | | Glycemia | 0.4239 | 1.528 | 1.125 | 2.076 |
| | | AST | 0.0585 | 1.060 | 1.034 | 1.087 |
| | | ALT | -0.0184 | 0.982 | 0.968 | 0.996 |
| | | Ferritin | 0.00112 | 1.001 | 1.000 | 1.002 |
| | | Platelets | -0.0105 | 0.990 | 0.982 | 0.997 |
| **2b** | **Significant fibrosis NASH-CRN F≥ 2** | | | | **Exp (β)** | **Exp (β)** |
| | | Intercept | -3.6323 | - | - | - |
| | | Weight | 0.0539 | 1.055 | 1.026 | 1.086 |
| | | Glycemia | 0.5166 | 1.676 | 1.131 | 2.485 |

(continued)

| Score | Diagnostic target | Variables | β coeff | Exp (β) | 95% CI inf | 95% CI sup |
|---|---|---|---|---|---|---|
| | | AST | 0.0695 | 1.072 | 1.044 | 1.101 |
| | | ALT | -0.0189 | 0.981 | 0.969 | 0.994 |
| | | Prothrombin index | -0.0594 | 0.942 | 0.905 | 0.981 |
| 3b | **Area of fibrosis** | | | | β | β |
| | | Intercept | 11.59720 | | 5.7242 3 | 17.47017 |
| | | Glycemia | 0.44087 | | 0.1611 8 | 0.72055 |
| | | AST | 0.04949 | | 0.0229 1 | 0.07606 |
| | | ALT | -0.02083 | | - 0.0366 1 | -0.00505 |
| | | Platelets | -0.00752 | | - 0.0164 1 | 0.00138 |
| | | Prothrombin index | -0.08903 | | - 0.1508 5 | -0.02721 |
| | | Hyaluronic acid | 0.02122 | | 0.0150 9 | 0.02736 |
| 4b | **Fractal dimension of fibrosis** | | | | β | β |
| | | Intercept | 0.74800 | | 0.5962 2 | 0.89978 |
| | | Weight | 0.00247 | | 0.0012 0 | 0.00374 |
| | | Glycemia | 0.02634 | | 0.0163 0 | 0.03637 |
| | | AST/ALT | 0.19838 | | 0.1278 8 | 0.26888 |
| | | Platelets | -0.0005524 | | - 0.0008 494 | - 0.000255 3 |
| | | Hyaluronic acid | 0.00035650 | | 0.0001 1211 | 0.000600 90 |
| | | | | | | |
| 5b | **Significant steatosis (rAOS ≥3%)** | | | | **Exp (β)** | **Exp (β)** |
| | | Intercept | -10.8186 | - | - | - |
| | | BMI | 0.2667 | 1.306 | 1.136 | 1.500 |
| | | Glycemia | 0.5154 | 1.674 | 1.047 | 2.678 |
| | | Triglycerides | 0.9728 | 2.645 | 1.388 | 5.042 |
| 6b | **Significant steatosis NASH-CRN grade≥ 1** | | | | Exp (β) | Exp (β) |
| | | Intercept | -11.1031 | - | - | - |
| | | BMI | 0.2124 | 1.237 | 1.108 | 1.380 |
| | | Glycemia | 0.6451 | 1.906 | 1.180 | 3.080 |
| | | Triglycerides | 0.7650 | 2.149 | 1.261 | 3.663 |
| | | Ferritin | 0.00149 | 1.001 | 1.000 | 1.003 |
| 7b | **Area of steatosis (log)** | | | | β | β |
| | | Intercept | -1.78517 | | - 2.9833 6 | -0.58698 |
| | | BMI | 0.08947 | | 0.0507 9 | 0.12815 |

(continued)

| Score | Diagnostic target | Variables | β coeff | Exp (β) | 95% CI inf | 95% CI sup |
|---|---|---|---|---|---|---|
| | | Glycemia | 0.09542 | | - 0.0215 8 | 0.21241 |
| | | Triglycerides | 0.22814 | | 0.0566 5 | 0.39963 |
| | | Ferritin | 0.0002852 | | - 0.0001074 | 0.0006778 |
| 8b | Fractal dimension of steatosis | | | | β | β |
| | | Intercept | 0.91010 | | 0.7236 3 | 1.09658 |
| | | BMI | 0.01502 | | 0.00900 | 0.02103 |
| | | Glycemia | 0.01625 | | - 0.00195 | 0.03446 |
| | | Triglycerides | 0.03534 | | 0.00865 | 0.06203 |
| | | Ferritin | 0.0000576 | | - 0.0000035 | 0.0001187 |
| 9b | NASH (NAS $\geq$ 3) | | | | Exp (β) | Exp (β) |
| | | Intercept | -7.5364 | - | - | - |
| | | BMI | 0.1596 | 1.173 | 1.059 | 1.300 |
| | | AST | 0.0479 | 1.049 | 1.024 | 1.075 |
| | | Ferritin | 0.00256 | 1.003 | 1.001 | 1.004 |
| 10b | NASH (NAS $\geq$ 5) | | | | | |
| | | Intercept | -2.4712 | - | - | - |
| | | Ferritin | 0.00148 | 1.001 | 1.001 | 1.002 |
| 11b | NASH (NAS 0-2/3-4/5-7) | | | | | |
| | | Intercept3 | -7.3898 | - | - | - |
| | | Intercept2 | -5.3943 | - | - | - |
| | | BMI | 0.1258 | 1.134 | 1.048 | 1.228 |
| | | AST | 0.0255 | 1.026 | 1.010 | 1.042 |
| | | Ferritin | 0.00158 | 1.002 | 1.001 | 1.003 |

### *Area and fractal dimension of fibrosis*

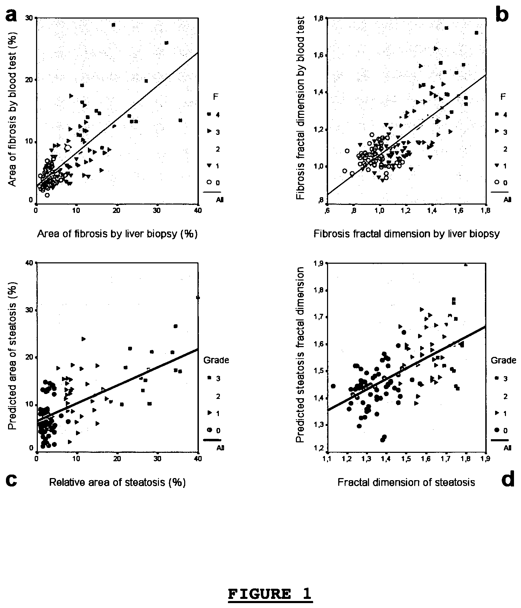

By multiple linear regression, the highest accuracy was obtained with a combination of the independent predictors of AOF (see tables 2 and 3). Thus, the correlation between AOF predicted by blood test and measured by liver biopsy was: $r_p$=0.731 (p<10$^{-3}$) **(figure 1a)**. The highest accuracy for fibrosis FD was obtained with a combination of the independent predictors (see tables 2 and 3). Thus, the correlation between fibrosis FD predicted by blood test and measured by liver biopsy was: $r_p$=0.734 (p<10$^{-3}$) **(figure 1b)**.

### Diagnosis of liver steatosis

### *Significant steatosis*

*Diagnostic performance* - Significant steatosis was defined in two ways. It was first defined by rAOS $\geq$ 3% since the plot area vs FD showed an inflexion at rAOS≈3% and FD≈1.4 **(figure 2).** The second definition was based on NASH-CRN grade $\geq$ 1. The prevalence of steatosis was significantly higher with rAOS $\geq$ 3%: 68.8% than with NASH-CRN grade $\geq$ 1: 54.7% (p<10$^{-3}$ by McNemar test).

Using binary logistic regression, significant steatosis defined by rAOS $\geq$ 3% was diagnosed by the independent variables as shown in **tables 2 and** 3. Diagnostic performance of this model was: AUROC: 0.797$\pm$0.027 (95%CI:

0.804-0.911), overall accuracy: 77.4%.

By using NASH-CRN grade $\geq 1$ as diagnostic target, the best score is shown in **tables 2 and 3.**

*Steatosis amount*

Using multiple linear regression, the rAOS could be estimated, with a moderate accuracy ($_aR^2=0.213$), by a score including blood and clinical variables as shown in **tables 2 and 3.** Thus, the correlation between rAOS predicted by blood test and measured by liver biopsy was: rp=0.647 (p<10$^{-3}$) **(figure 1c)**.

*Fractal dimension of steatosis*

Using multiple linear regression, the steatosis FD could be estimated, with a moderate accuracy ($_aR^2=0.247$), by a score including blood and clinical variables as shown in **tables 2** and 3. Thus, the correlation between steatosis FD predicted by blood test and measured by liver biopsy was: $r_p=0.621$ (p<10$^{-3}$) **(figure 1d)**.

**Diagnosis of NASH**

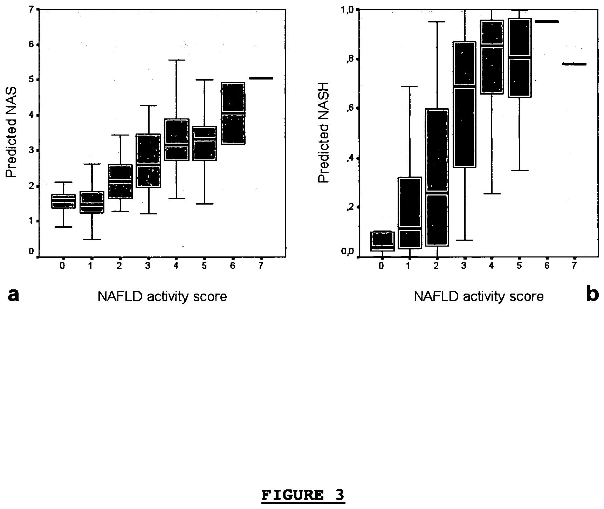

NASH was independently predicted as shown in tables 2 and 3 with corresponding AUROC. The correlation between NASH predicted by blood test and NAS measured by liver biopsy was: $r_s=0.726$ (p<10$^{-3}$) **(figure 3b).**

**Relationship between lesions**

In order to validate the blood tests, we evaluated the ability of those tests to reflect the relationships between lesions.

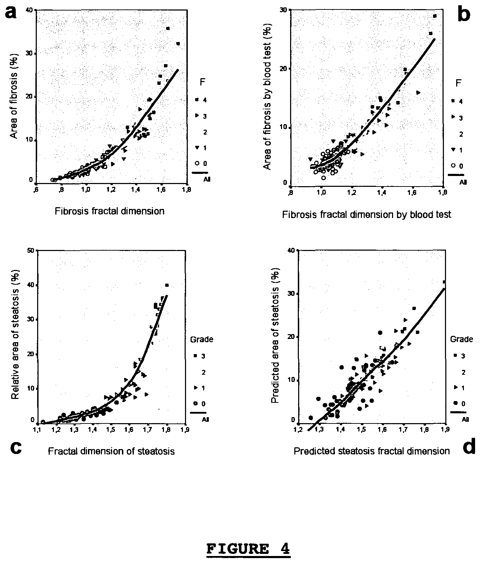

*Area and FD -* The exponential relationship between histological AOF and FD ($r_s=0.971$, p<10$^{-3}$) was well reflected by the relationship between respective blood tests ($r_s=0.861$, p<10$^{-3}$) **(figure 4a and b).**

The exponential relationship between histological rAOS and steatosis FD ($r_s=0.976$, p<10$^{-3}$) was well reflected by the relationship between respective blood tests ($r_s=0.886$, p<10$^{-3}$) **(figure 4c and d).**

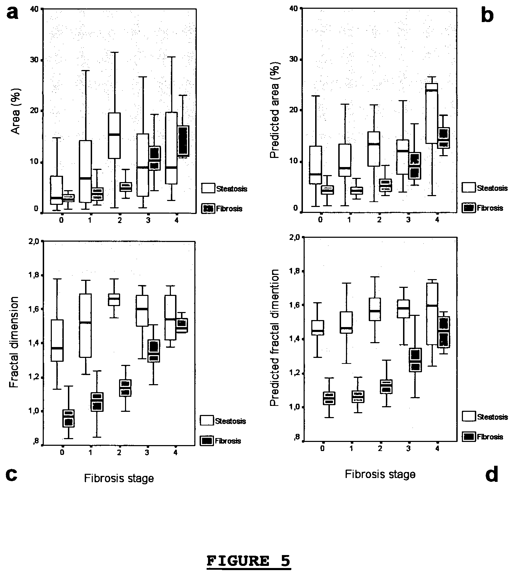

*Fibrosis and steatosis -* NASH-CRN fibrosis stage is considered here as a chronological reference since it is assumed to increase as a function of time. AOS peaked in NASH-CRN fibrosis stage 2 with a quadratic curve whereas AOF almost linearly increased as a function of fibrosis stage **(figure 5a)**. These relationships were well reflected by respective blood tests except for AOS in F4 but there were a few patients **(figure 5b)**. The same curves were observed with FD of fibrosis or steatosis **(figure 5c)** whereas they were well reflected by respective blood tests **(figure 5d).**

**MRI Studies**

21 patients with steatohepatitis were included, along with two controls without steatohepatitis.

Liver biopsy was performed in order to diagnose alcoholic or non-alcoholic steatohepatitis.

Steatosis degree was evaluated by an expert using steatosis grading

- S0 (0% of hepatocytes),
- S1 (less than 10%),
- S2 (between 10% and 30%),
- S3 (between 30% and 50%) and
- S4 (more than 50%).

Steatosis area (AOS) (in percentage) was computed by our automatic image analysis method, previously described in the present application, which measures the area of lipid vacuoles presented in hepatocytes. Quantification methods by MRI, already described in the literature, were implemented, namely: Hussain, Fast Spin Echo with and without fat saturation (FSE), 2-point Dixon and 3-point Dixon.

An additional method based on multi-echo gradient-echo MRI (MFGRE) was developed, as previously described in the present application, and applied.

Correlations were evaluated by Spearman correlation coefficient (Rs) and intraclass correlation coefficient (Ric).

**Results**

The distribution of our population by steatosis grading was:

- S0 = 2 patients,
- S1 = 4 patients,
- S2 = 4 patients,
- S3 = 7 patients and
- S4 = 6 patients.

*Correlation between steatosis grading and AOS:*

AOS was correlated with steatosis grades (Rs = 0.82 and p<0.001). AOS was significantly different between all steatosis grades (p<0.001), e.g. between S0 to S2 grades (less than 30%) and S3 to S4 grades (more than 30%), respectively: $4.2 \pm 2.4$ versus $16.4 \pm 8.9$ (p<0.001).

*Correlation between MRI quantification methods and steatosis grading:* Intraclass correlation between steatosis grades and MRI quantification methods was Rs = 0.66 for MFGRE, Rs = 0.69 for Hussain, Rs = 0.80 for FSE; Rs = 0.68 for 2-point Dixon and Rs = 0.73 for 3-point Dixon (p<0.001 for all correlations).

*Correlation between MRI quantification and AOS:*

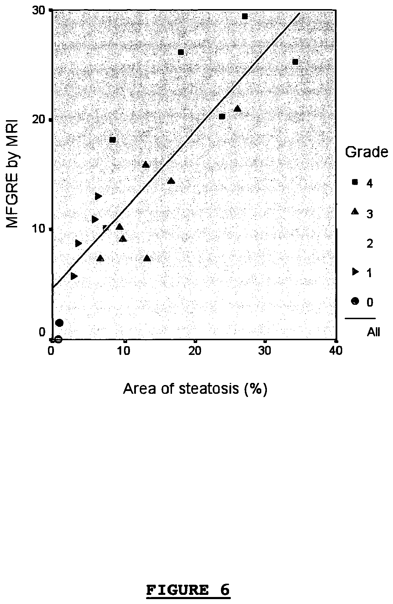

Steatosis quantification by MFGRE method correlated best to AOS (Rs = 0.77 and Ric = 0.85) (see Figure 6).

Correlation coefficients obtained by other methods were, respectively: 0.71 and 0.57 for Hussain, 0.80 and 0.61 for FSE; 0.71 and 0.55 for 2-point Dixon; 0.61 and 0.28 for 3-point Dixon.

In multiple linear regression, MFGRE was the only MRI quantification method independently linked to AOS.

## Claims

**1.** An in-vitro non-invasive method for quantifying the lesions of the liver of a patient comprising:

measuring the following biomarkers in a sample of the patient: platelets, AST (aspartate aminotransferase), ALT (alanine aminotransferase), ferritin and
glycemia and measuring the following clinical markers: age and weight, and
combining said measures in a logistic function.

**2.** The method according to claim **1,** wherein the lesion is due or related to liver impairment, liver fibrosis, liver steatosis, non-alcoholic fatty liver disease (NAFLD), steato-hepatitis, or non-alcoholic steato-hepatitis (NASH).

## Patentansprüche

**1.** Nichtinvasives In-vitro-Verfahren zur Quantifizierung von Läsionen der Leber eines Patienten, das Folgendes umfasst:

Messen der folgenden Biomarker in einer Probe des Patienten: Thrombozyten, AST (Aspartat-Aminotransferase), ALT (Alanin-Aminotransferase), Ferritin und Glykämie, und Messen der folgenden klinischen Marker: Alter und Gewicht, und Kombinieren der Messwerte in einer logistischen Funktion.

**2.** Verfahren nach Anspruch **1,** wobei die Läsion aufgrund von Leberfunktionsstörung, Leberfibrose, Lebersteatose, nichtalkoholischer Fettlebererkrankung (NAFLD), Steatohepatitis oder nichtalkoholischer Steatohepatitis (NASH) ist oder damit zusammenhängt.

## Revendications

**1.** Une méthode in vitro non invasive pour quantifier les lésions du foie d'un patient comprenant :

la mesure des biomarqueurs suivants dans un échantillon du patient : plaquettes, AST (aspartate aminotransférase), ALT (alanine aminotransférase), ferritine et glycémie et la mesure des marqueurs cliniques suivants : âge et poids, et la combinaison desdites mesures dans une fonction logistique.

2. La méthode selon la revendication **1,** dans laquelle la lésion est causée ou liée à une insuffisance du foie, une fibrose du foie, une stéatose du foie, une stéatose hépatique non alcoolique, une stéatohépatite, ou une stéatohépatite non alcoolique.

**FIGURE 1**

FIGURE 2

a          NAFLD activity score

b          NAFLD activity score

**FIGURE 3**

**FIGURE 4**

**FIGURE 5**

**FIGURE 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005116901 A
- WO 2006082522 A, Poynard
- WO 2008021192 A, Watkins

**Non-patent literature cited in the description**

- **CALES P et al.** *J Hepatol,* 2009, vol. 50, 165-173
- **CALES P et al.** *Clin Biochem,* 2008, vol. 41, 10-18
- **KLEINER DE et al.** *Hepatology,* 2005, vol. 41, 1313-1321
- *Hepatology,* 1994, vol. 20, 15-20
- **ROULLIER V et al.** *Conf Proc IEEE Eng Med Biol Soc,* 2007, vol. 2007, 5575-5578
- **BOURSIER J et al.** *J Hepatol,* 2009, vol. 50, S357
- **PILETTE C et al.** *J Hepatol,* 1998, vol. 28, 439-446
- **MOAL F et al.** *Hepatology,* 2002, vol. 36, 840-849
- **BORKOWF CB et al.** *Stat Med,* 2003, vol. 22, 1477-1493